## Europäisches Patentamt
## European Patent Office
## Office européen des brevets

(19)

(11) Veröffentlichungsnummer: **0 035 102 B1**

(12) # EUROPÄISCHE PATENTSCHRIFT

(45) Veröffentlichungstag der Patentschrift:
**19.10.83**

(51) Int. Cl.³: **C 07 C 103/52**, A 61 K 35/14, A 61 K 35/28, C 07 G 7/00

(21) Anmeldenummer: **81100268.2**

(22) Anmeldetag: **15.01.81**

(54) **Aus gesunden weissen Blutkörperchen beziehungsweise Granulozyten isolierbares, das Wachstum beziehungsweise die Vermehrung von normalen und leukämischen myeloiden Zellen selektiv hemmendes Oligopeptid, Verfahren zu dessen Herstellung und dieses enthaltende Arzneimittel.**

(30) Priorität: **15.01.80 HU RI000068**

(43) Veröffentlichungstag der Anmeldung:
**09.09.81 Patentblatt 81/36**

(45) Bekanntmachung des Hinweises auf die Patenterteilung:
**19.10.83 Patentblatt 83/42**

(84) Benannte Vertragsstaaten:
**AT BE CH DE FR GB IT LI NL SE**

(56) Entgegenhaltungen:
**US-A-3 937 816**
**US-A-3 973 001**
**Chemical Abstracts Band 78, Nr. 2, 1973 Columbus, Ohio, USA BALAZS et al. »Preparations selectively hindering the proliferation of tumor cells« Seite 241, rechte Spalte, Abstract Nr. 7819t**

(73) Patentinhaber: **RICHTER GEDEON VEGYESZETI GYAR R.T., Gyömröi ut 19-21, H-1475 Budapest X (HU)**

(72) Erfinder: **Balázs, András, Dr., Mária tér 2, Budapest I. (HU)**
Erfinder: **Sajgó, Mihály, Dr., Deák F. u. 52, Budapest XIX (HU)**
Erfinder: **Kisfaludy, Lajos, Dr., Riadó u. 6/a, Budapest II (HU)**
Erfinder: **Klupp, Tibor, Zöldlomb u. 19/a, Budapest II. (HU)**
Erfinder: **Barabás, Miklósné, Szalag u. 13, Budapest I (HU)**

(74) Vertreter: **Beszédes, Stephan G. Dr., Münchener Strasse 80a Postfach 1168, D-8060 Dachau (DE)**

**0 035 102**

Aus gesunden weißen Blutkörperchen beziehungsweise Granulozyten
isolierbares, das Wachstum beziehungsweise die Vermehrung von normalen und
leukämischen myeloiden Zellen selektiv hemmendes Oligopeptid,
Verfahren zu dessen Herstellung und dieses enthaltende Arzneimittel

Die Erfindung betrifft ein aus gesunden weißen Blutkörperchen beziehungsweise Granulozyten isolierbares, das Wachstum beziehungsweise die Vermehrung von normalen und leukämischen myeloiden Zellen selektiv hemmendes Oligopeptid, ein Verfahren zu dessen Herstellung und dieses enthaltende Arzneimittel.

Bekanntlich sind fast sämtliche in der Chemotherapie der bösartigen beziehungsweise malignen Tumore verwendeten cytostatischen Wirkstoffe, wie die Alkyliermittel, die Antimetaboliten oder die Indolalkaloide, stark toxische Produkte, welche auf alle sich teilenden Zelltypen schädliche Wirkungen ausüben. Demgegenüber sind die bösartigen beziehungsweise malignen Tumore pathologische Veränderungen eines einzigen Zelltyps; deshalb wäre es erwünscht, selektiv wirkende Mittel zur Hemmung ihres Wachstums beziehungsweise ihrer Vermehrung zu schaffen und anzuwenden (vergleiche S. Eckhardt, Klinische Onkologie, Medicina-Verlag, Budapest, 1977).

Zur Feststellung von solchen das Wachstum beziehungsweise die Vermehrung der Tumorzellen spezifisch hemmenden Mitteln wurden bereits bisher weitläufige Forschungsarbeiten durchgeführt; es wurden unter anderem etwa 25 verschiedene aus einheitlichen Zellgeweben hergestellte Zellenextrakte beschrieben, von denen behauptet beziehungsweise für die nachgewiesen wurde, daß sie bestimmte Zelltypen selektiv hemmende natürliche Regulatoren enthalten (vergleiche A. Balázs, I. Blazsek: Control of Cell Proliferation by Endogenous Inhibitors, Akadémiai Könyvkiadó, Budapest und Elsevier, North Holland, 1979). Gute Ergebnisse wurden besonders mit Extrakten von weißen Blutkörperchen beziehungsweise Granulozyten erzielt (vergleiche Rytömaa und Kiviniemi, Cell Tissue Kinet. 1 [1968], 341; Paukovits, Nat. Cancer Inst. Monogr. 38 [1973], 147; Paukovits und Mitarbeiter, Oncology 34 [1977], 187).

Die biologische Wirksamkeit von teilweise gereinigten Extrakten von weißen Blutkörperchen beziehungsweise Granulozytenextrakten wurde auch an an Leukämie leidenden Patienten demonstriert (vergleiche Rytömaa und Mitarbeiter, Scand. J. Haematol., Suppl. 27 [1976], 5); schon früher wurden von A. Balázs und Mitarbeiter (ungarische Patentschrift 162 447) aus menschlichem Blut beziehungsweise aus Kalbsblut oder aus der Bauchfellflüssigkeit beziehungsweise peritonealen Flüssigkeit von Menschen beziehungsweise Kälbern teilweise gereinigte Extrakte von weißen Blutkörperchen beziehungsweise Granulozytenextrakte hergestellt, welche eine selektive Hemmwirkung gegen die Vermehrung von weißen Blutkörperchen zeigten. Sämtliche bisher beschriebenen beziehungsweise hergestellten derartigen Extrakte waren aber nur teilweise gereinigt, noch zahlreiche Begleitstoffe enthaltende Präparate; die Herstellung von das Wachstum beziehungsweise die Vermehrung von bestimmten Zelltypen selektiv hemmenden reinen Wirkstoffen war dagegen bisher nicht bekannt.

Der Erfindung liegt daher die Aufgabe zugrunde, einen aus weißen Blutkörperchen beziehungsweise Granulozyten isolierbaren, das Wachstum beziehungsweise die Vermehrung der normalen und leukämischen myeloiden Zellen selektiv hemmenden reinen neuen Wirkstoff, ein Verfahren zu dessen Herstellung und diesen enthaltende Arzneimittel zu schaffen.

Das obige wurde überraschenderweise durch die Erfindung erreicht.

Bei der Weiterentwicklung der Forschungsarbeit wurde nämlich nun überraschenderweise festgestellt, daß aus Extrakten von aus tierischem oder menschlichem Blut, besonders aus Pferdeblut, gewonnenen weißen Blutkörperchen oder von aus Granulozyte enthaltenden tierischen Organen, besonders Kalbsmilz, durch spezielle Fraktionierungsverfahrensweisen ein chemisch praktisch reiner einheitlicher gut charakterisierbarer Oligopeptidwirkstoff isoliert werden kann, welcher schon in äußerst niedrigen Konzentrationen eine signifikante selektive Hemmwirkung gegen das Wachstum beziehungsweise die Vermehrung von normalen und leukämischen myeloiden Zellen zeigt.

Gegenstand der Erfindung ist daher ein aus gesunden weißen Blutkörperchen beziehungsweise Granulozyten isolierbares, das Wachstum beziehungsweise die Vermehrung von normalen und leukämischen myeloiden Zellen selektiv hemmendes Oligopeptid, welches dadurch gekennzeichnet ist, daß es die summenmäßige Aminosäurezusammensetzung

$Tau_1, Asx_1, Ser_2, Thr_1, Glx_3, Gly_2, Ala_1, (PO_4)_1,$

worin

Tau für einen Rest von Taurin steht,
Asx einen Rest von L-Asparagin und/oder L-Asparaginsäure bedeutet,
Ser einen Rest von L-Serin darstellt,
Thr für einen Rest von L-Threonin steht,
Glx einen Rest von L-Glutamin und/oder L-Glutaminsäure bedeutet,
Gly einen Rest von Glykokoll darstellt und

2

Ala für einen Rest von L-Alanin steht,

aufweist und bei einem pH-Wert von 6,5 eine negative Ladung und eine auf Asparaginsäure bezogene relative elektrophoretische Beweglichkeit von −0,55 bis −0,65 beziehungsweise bei einem pH-Wert von 1,9 keine Ladung und eine auf ε-(Dinitrophenyl)-lysin [ε-Dnp-Lysin] bezogene relative Beweglichkeit von etwa 0,26 zeigt und auf Chlortolidin positiv reagiert.

Die oben verwendeten Abkürzungen wurden von der IUPAC (vergleiche Journal of Biological Chemistry 247 [1972], 977) festgesetzt.

Bekanntlich sind die Granulozyten der in den weißen Blutkörperchen (Leukozyten) überwiegend enthaltene Haupttyp derselben.

Beim erfindungsgemäßen chemisch einheitlichen Oligopeptid hat die Peptidkette nach den durchgeführten analytischen Untersuchungen keine freie endständige Aminogruppe.

Gegenstand der Erfindung ist auch ein Verfahren zur Herstellung des erfindungsgemäßen Oligopeptides, welches dadurch gekennzeichnet ist, daß

a) aus tierischem oder menschlichem Blut, insbesondere Pferdeblut, gewonnene gesunde weiße Blutkörperchen in einer Pufferlösung mit einem pH-Wert von 7 bis 8 homogenisiert werden, das flüssige Homogenisat zentrifugiert wird, aus der überstehenden Flüssigkeit die gelösten Bestandteile mit Molekulargewichten unter 10 000 abgetrennt werden, diese Fraktion einer weiteren Fraktionierung, zweckmäßig durch Gelchromatographie, unterworfen wird und die sich

bei den Volumwerten $\frac{V_e}{V_o}$ von 1,15 bis 1,45 abscheidende Fraktion abgetrennt und gegebenenfalls lyophilisiert wird oder

b) Granulozyten enthaltende tierische Organe, insbesondere Kalbsmilz, in einem mit Wasser mischbaren organischen Lösungsmittel homogenisiert werden, aus der erhaltenen flüssigen Suspension die Feststoffe abgetrennt und mit einem fettlösenden organischen Lösungsmittel extrahiert werden, die so erhaltenen Feststoffe mit Wasser extrahiert werden, aus der erhaltenen flüssigen Suspension der unlösliche Teil abgetrennt wird und aus der flüssigen Phase die gelösten Bestandteile mit Molekulargewichten unter 10 000 abgetrennt werden, diese Fraktion einer weiteren Fraktionierung, zweckmäßig durch Gelchromatographie, unterworfen wird und die sich

bei den Volumenwerten $\frac{V_e}{V_o}$ von 1,3 bis 2,5 abscheidende Fraktion abgetrennt und gegebenenfalls lyophilisiert wird

und das nach einer der obigen Varianten a) und b) erhaltene Wirkstoffkonzentrat (im folgenden »Fraktion GI-3« genannt) durch ein auf Grund von Ionenladung und Teilchengröße fraktionierendes Chromatographieren und/oder durch Papier-Elektrophorese gereinigt wird und das bei einem pH-Wert von 6,5 eine negative Ladung und eine auf Asparaginsäure bezogene relative Beweglichkeit von −0,55 bis −0,65 beziehungsweise bei einem pH-Wert von 1,9 keine Ladung und eine auf ε-(Dinitrophenyl)-lysin bezogene relative Beweglichkeit von etwa 0,26 zeigende und auf Chlortolidin positiv reagierende Oligopeptid abgetrennt und gegebenenfalls lyophilisiert wird.

Vorteilhaft wird als Pufferlösung eine Phosphatpufferlösung, beispielsweise aus Dinatriumhydrogenphosphat und Kaliumdihydrogenphosphat, verwendet.

Zweckmäßig wird das Abtrennen der Fraktion mit Molekulargewichten unter 10 000 durch Molekularfiltration durchgeführt.

Vorzugsweise wird als mit Wasser mischbares organisches Lösungsmittel Aceton verwendet.

Zweckmäßig wird das Abtrennen der in den flüssigen Suspensionen enthaltenen Feststoffe beziehungsweise unlöslichen Teile durch Zentrifugieren durchgeführt.

Vorzugsweise werden als fettlösende organische Lösungsmittel Chlorkohlenwasserstoff verwendet.

Bei der Variante a) des erfindungsgemäßen Verfahrens kann vorteilhaft eine Gewinnung eines Granulozytenniederschlages durch Suspendieren des, vorteilhaft durch Behandlung des tierischen beziehungsweise menschlichen Blutes mit Heparin, Sedimentieren und Abtrennen der an weißen Blutkörperchen (Leukozyten) reichen Schicht und Zentrifugieren erhaltenen, an Granulozyten angereicherten Niederschlages in der Pufferlösung mit einem pH-Wert von 7 bis 8 und Zentrifugieren vorgeschaltet werden, welcher Granulozytenniederschlag dann den für die Variante a) angegebenen Maßnahmen unterworfen wird.

Gegebenenfalls kann auch die die Bestandteile mit Molekulargewichten unter 10 000 enthaltende Fraktion vor ihrer weiteren Fraktionierung lyophilisiert werden.

Bei der Variante b) des erfindungsgemäßen Verfahrens können die mit dem mit Wasser mischbaren organischen Lösungsmittel homogenisierten tierischen Organe vor der Abtrennung der Feststoffe aus ihnen bebrütet werden.

Ferner sind erfindungsgemäß Arzneimittel, welche das erfindungsgemäße Oligopeptid als Wirkstoff, zweckmäßigerweise zusammen mit üblichen pharmazeutischen Konfektionierungsmitteln, enthalten, vorgesehen.

Das zweckmäßig in lyophilisierter Form isolierte, erfindungsgemäße Oligopeptid zeigt nämlich wie bereits erwähnt eine vorteilhaft selektiv hemmende Wirkung in bezug auf das Wachstum beziehungsweise die Vermehrung von normalen und leukämischen myeloiden Zellen. Es hemmt spezifisch nur das Wachstum beziehungsweise die Vermehrung von myeloiden Zellen (von granuloiden Zellen von Ratten und Mäusen und transplantablen tierischen beziehungsweise spontanen akuten menschlichen myeloid-leukämischen Zellen), ist aber gegen die normalen Thymozyten, von Phytohämagglutinin stimulierten (PHA-stimulierten) Lymphozyten, subakuten lymphoiden leukämischen Thymozyten, menschlichen lymphoiden leukämischen Blutzellen und HeLa humanen HeLa-Tumorzellen (aus menschlichen Cervix-Karzinomen isolierten, und in vitro unbeschränkt vermehrungsfähigen handelsüblichen Zellenstrang, der erstmalig 1952 von Gey isoliert wurde) wirkungslos.

Die als Zwischenprodukt des erfindungsgemäßen Verfahrens erhältliche partiell gereinigte Fraktion GI-3 enthält natürlich ebenfalls schon den erfindungsgemäßen Oligopeptidwirkstoff, aber in nicht isolierter Form in Begleitung von verschiedenen weniger aktiven Bestandteilen und hauptsächlich einer überwiegenden Menge von inaktiven Verunreinigungen. Da aber die eigentliche aktive Substanz, der Oligopeptidwirkstoff in diesem Zwischenprodukt schon in erheblichen Mengen zugegen ist, zeigt es ebenfalls bereits eine (dem niedrigeren Oligopeptidgehalt entsprechend niedrigere) spezifische Hemmwirkung gegen das Wachstum beziehungsweise die Vermehrung von myeloiden Zellen. So ist in vitro die wirksame Mindestdosis (MED) der Fraktion GI-3 bezüglich der Hemmung des Einbaues von $^3$H-Thymidin in die säureunlösliche Desoxyribonucleinsäure (DNS) 110 µg/cm$^3$ und bezüglich der sich in Agar-Gel bildenden Kolonien 8 µg/cm$^3$. Die entsprechenden ED$_{50}$-Werte sind 430 µg/cm$^3$ beziehungsweise 90 µg/cm$^3$.

Der erfindungsgemäße Oligopeptidwirkstoff zeigt die folgenden charakteristischen Eigenschaften:

Wirkungsdauer:  in Suspensionskulturen  > 8 Stunden
                in Gelkolonien          > 1 Woche.

Er ist nicht toxisch; so verursacht er im »$^{51}$Cr-release test« keine Verminderung der Lebenskraft beziehungsweise Vitalität der Zellen. Der Angriffspunkt der Wirkung ist die Phase G$_1$ des Zellenzyklus.

Die Wärmebeständigkeit des erfindungsgemäßen Oligopeptides ist wie folgt. Durch eine 60 Minuten lange Wärmebehandlung bei 80°C wird seine biologische Wirksamkeit nicht beeinflußt und auch die Spezifizität der Wirkung bleibt unverändert. In Lösung bleibt die Wirkung bei 37°C nach 72 Stunden unverändert. In der Lyophilisatform kann der erfindungsgemäße Oligopeptidwirkstoff bei −20°C mindestens 7 Wochen und in der Form von aus Aceton oder Chloroform gefälltem Pulver bei +4°C mindestens 18 Monate ohne Wirkungsverlust gelagert werden.

Die zur Ermittlung der Wirksamkeit des durch Ionenaustausch-Chromatographie beziehungsweise Papier-Elektrophorese isolierten und rückgetesteten erfindungsgemäßen Oligopeptidwirkstoffes sowie zum Vergleich der Wirksamkeiten des reinen erfindungsgemäßen Oligopeptidwirkstoffes und der Fraktion GI-3 in vitro durchgeführten Versuche lieferten die in den folgenden Tabellen 1 und 2 zusammengestellten Ergebnisse.

Tabelle 1

Selektive Wirkung von 100 µg/cm$^3$ des reinen erfindungsgemäßen Oligopeptidwirkstoffes auf den Einbau von $^3$H-Thymidin in die säureunlösliche Desoxyribonucleinsäure in Knochenmark- und Thymus-drüsenkulturen

| Versuchs-gruppe | Zahl der Versuche | Hemmung im Knochenmark | | Thymus in der Hemmungsdrüse | |
|---|---|---|---|---|---|
| | | in % der beim Blindversuch | Signifikanz P | in % der beim Blindversuch | Signifikanz P |
| 1 | 12 | 53,2 | | 14,6 | |
| | | | <0,001 | | >0,2 |
| 2 | 14 | 59,9 | | 7,3 | |

Tabelle 2

Dosis/Wirkung-Zusammenhänge beim reinen erfindungsgemäßen Oligopeptidwirkstoff und bei der Fraktion GI-3

| Art der Wirkung | Reiner erfindungsgemäßer Oligopeptidwirkstoff | | Fraktion GI-3 | |
| --- | --- | --- | --- | --- |
| | Wirksame Mindestdosis (MED) in Picomol/cm$^3$ | ED$_{50}$-Wert in Picomol/cm$^3$ | Wirksame Mindestdosis (MED) in μg/cm$_3$ | ED$_{50}$-Wert in μg/cm$^3$ |
| Einbau von $^3$H-Thymidin in Knochenmark-Suspensionskulturen, 3 bis 4 Stunden | 2,5*) | 7,8*) | 110*) | 430*) |
| Kolonienbildung in der Agar-gel-Kapillare, 7 Tage | 0,2**) | 1,6**) | 88*) | 90*) |

*) = Meßwerte.
**) = Berechnete Werte.

Das erfindungsgemäße Oligopeptid kann als biologischer Wirkstoff zur Hemmung der Wucherung beziehungsweise Proliferation von humanen normalen beziehungsweise leukämischen Knochenmark- und Blutzellen in vitro in Konzentrationen von 0,2 bis 10,0 Picomol/cm$^3$ verwendet werden.

Die Erfindung wird an Hand der folgenden Beispiele näher erläutert.

### Beispiel 1

### Herstellung der Fraktion GI-3 aus Pferdeblut

Es wurden 10 l Pferdeblut mit 3500 Einheiten/l Heparin behandelt und dann zur Sedimentierung 30 Minuten bei Raumtemperatur stehengelassen. Die an weißen Blutkörperchen (Leukozyten) reiche obere Schicht wurde abgetrennt und mit einer Kraft von 800 g zentrifugiert. Die als Niederschlag erhaltene 80% Granulozyten enthaltende Zellenpopulation wurde dann in 200 cm$^3$ einer 0,06 m Phosphatpufferlösung mit einem pH-Wert von 7,4 (Zusammensetzung: 9,500 g Dinatriumhydrogenphosphatdihydrat und 1,815 g Kaliumdihydrogenphosphat in 1 l destilliertem Wasser) suspendiert und wieder mit einer Kraft von 800 g zentrifugiert. Der erhaltene Granulozytenniederschlag wurde in derselben Phosphatpufferlösung in einer Konzentration von 4 · 10$^9$ Zellen/cm$^3$ suspendiert und in einer Potterschen Homogenisiervorrichtung mit 10 Hüben/Minute homogenisiert.

Das erhaltene Homogenisat wurde mit einer Kraft von 1550 g zentrifugiert und die abgetrennte überstehende Flüssigkeit wurde einer Ultrafiltration durch eine Membrane aus Amicon PM 10® unter einem Druck von 3 at unterworfen. Das erhaltene Filtrat wurde lyophilisiert und das Lyophilisat wurde in einer 0,05 m Ammoniumbicarbonatpufferlösung (pH-Wert = 7,9) gelöst und an einer Säule von Sephadex G 10® gelchromatographiert. Die bei den Volumwerten $\frac{V_e}{V_o}$ von 1,15 bis 1,45 abgeschiedene Fraktion wurde abgetrennt und lyophilisiert. So wurden 65 mg Wirkstoffkonzentrat, das heißt Fraktion GI-3, in Form eines weißen Pulvers erhalten.

### Beispiel 2

### Herstellung der Fraktion GI-3 aus Kalbsmilz

Es wurden 1000 g gereinigte und zerkleinerte Kalbsmilz mit auf 4°C gekühltem Aceton bis zu einem Gesamtvolumen von 4 l versetzt, das Gemisch wurde bei dieser Temperatur homogenisiert, 60 Minuten lang bebrütet und dann zentrifugiert. Die abgetrennten Feststoffe wurden mit Aceton gewaschen und unter Vakuum bei 20°C getrocknet.

Die trockenen Feststoffe wurden 30 Minuten lang mit 2 l Chloroform extrahiert und die dadurch angefallenen Feststoffe wurden abgetrennt, bei Raumtemperatur getrocknet, 16 Stunden mit 3 l

doppeldestilliertem Wasser gerührt und dann mit einer Kraft von 20 000 g zentrifugiert. Die überstehende Flüssigkeit wurde lyophilisiert und das Lyophilisat wurde in einer 0,05 m Ammoniumbicarbonatpufferlösung (pH-Wert = 7,9) gelöst und an einer Säule von Sephadex G 15®

gelchromatographiert. Die bei den Volumwerten $\frac{V_e}{V_o}$ von 1,3 bis 2,5 abgeschiedene Fraktion wurde abgetrennt und lyophilisiert. So wurden 3,54 g Wirkstoffkonzentrat, das heißt Fraktion GI-3, in Form eines weißen Pulvers erhalten.

Beispiel 3

Herstellung des reinen erfindungsgemäßen Oligopeptidwirkstoffes

Es wurden 300 mg der wie im Beispiel 1 oder 2 beschrieben hergestellten Fraktion GI-3 auf ein Chromatographierpapier (Whatman 3MM®) aufgetragen; der Ausgangspunkt wurde in der Mitte des Blattes angeordnet und die Gesamtmenge der aufgetragenen Probe wurde in einer Länge von 30 cm verteilt. Die Elektrophorese wurde in einem Puffergemisch aus Pyridin, Essigsäure und Wasser im Volumverhältnis von 90 : 4 : 900 (pH-Wert = 6,5) in einem Elektrophoreseapparat mit horizontaler Anordnung, mit einem Spannungsgradienten von 50 V/cm und mit einer Zeitdauer von 2 Stunden durchgeführt. Die Lage des sauren, auf Ninhydrin negativ und auf Chlor positiv reagierenden Bestandteiles wurde durch Färben mit Chlortolidin ermittelt. Der auf diese Weise erhaltene aktive Bestandteil wurde mit einem Puffergemisch aus Essigsäure, Ameisensäure und Wasser im Volumverhältnis von 8 : 2 : 90 (pH-Wert = 1,9) von dem Papier eluiert und im selben Puffergemisch einer weiteren Elektrophorese mit einem Spannungsgradienten von 80 V/cm und mit einer Zeitdauer von 90 Minuten unterworfen. Die Lage des bei diesem pH-Wert keine Ladung zeigenden aktiven Bestandteiles wurde mit Hilfe der Chlortolidinreaktion ermittelt. Dieser aktive Bestandteil wurde mit demselben Puffergemisch (pH-Wert = 1,9) eluiert und das Eluat wurde lyophilisiert. So wurden 8 µg reiner erfindungsgemäßer Oligopeptidwirkstoff in Form eines weißen Pulvers erhalten.

**Patentansprüche für die Vertragsstaaten: BE, CH, DE, FR, GB, IT, LI, NL, SE**

1. Aus gesunden weißen Blutkörperchen beziehungsweise Granulozyten isolierbares, das Wachstum beziehungsweise die Vermehrung von normalen und leukämischen myeloiden Zellen selektiv hemmendes Oligopeptid, dadurch gekennzeichnet, daß es die summenmäßige Aminosäure- zusammensetzung

$Tau_1$, $Asx_1$, $Ser_2$, $Thr_1$, $Glx_3$, $Gly_2$, $Ala_1$, $(PO_4)_1$,

worin

Tau für einen Rest von Taurin steht,
Asx einen Rest von L-Asparagin und/oder L-Asparaginsäure bedeutet,
Ser einen Rest von L-Serin darstellt,
Thr für einen Rest von L-Threonin steht,
Glx einen Rest von L-Glutamin und/oder L-Glutaminsäure bedeutet,
Gly einen Rest von Glykokoll darstellt und
Ala für einen Rest von L-Alanin steht,

aufweist und bei einem pH-Wert von 6,5 eine negative Ladung und eine auf Asparaginsäure bezogene relative elektrophoretische Beweglichkeit von −0,55 bis −0,65 beziehungsweise bei einem pH-Wert von 1,9 keine Ladung und eine auf ε-(Dinitrophenyl)-lysin bezogene relative Beweglichkeit von etwa 0,26 zeigt und auf Chlortolidin positiv reagiert.

2. Verfahren zur Herstellung des Oligopeptides nach Anspruch 1, dadurch gekennzeichnet, daß man

a)  aus tierischem oder menschlichem Blut, insbesondere Pferdeblut, gewonnene gesunde weiße Blutkörperchen in einer Pufferlösung mit einem pH-Wert von 7 bis 8 homogenisiert, das flüssige Homogenisat zentrifugiert, aus der überstehenden Flüssigkeit die gelösten Bestandteile mit Molekulargewichten unter 10 000 abtrennt, diese Fraktion einer weiteren Fraktionierung, zweckmäßig durch Gelchromatographie, unterwirft und die sich bei den Volumwerten $\frac{V_e}{V_o}$ von 1,15 bis 1,45 abscheidende Fraktion abtrennt und gegebenenfalls lyophilisiert oder

b)  Granulozyten enthaltende tierische Organe, insbesondere Kalbsmilz, in einem mit Wasser mischbaren organischen Lösungsmittel homogenisiert, aus der erhaltenen flüssigen Suspension die Feststoffe abtrennt und mit einem fettlösenden organischen Lösungsmittel extrahiert, die so erhaltenen Feststoffe mit Wasser extrahiert, aus der erhaltenen flüssigen Suspension den unlöslichen Teil abtrennt und aus der flüssigen Phase die gelösten Bestandteile mit Molekulargewichten unter 10 000 abtrennt, diese Fraktion einer weiteren Fraktionierung, zweckmäßig durch Gelchromatographie, unterwirft und die sich bei den Volumwerten $\frac{V_e}{V_o}$ von 1,3 bis 2,5 abscheidende Fraktion abtrennt und gegebenenfalls lyophilisiert

und das nach einer der obigen Varianten a) und b) erhaltene Wirkstoffkonzentrat durch ein auf Grund von Ionenladung und Teilchengröße fraktionierendes Chromatographieren und/oder durch Papier-Elektrophorese reinigt und das bei einem pH-Wert von 6,5 eine negative Ladung und eine auf Asparaginsäure bezogene relative Beweglichkeit von −0,55 bis −0,65 beziehungsweise bei einem pH-Wert von 1,9 keine Ladung und eine auf ε-(Dinitrophenyl)-lysin bezogene relative Beweglichkeit von etwa 0,26 zeigende und auf Chlortolidin positiv reagierende Oligopeptid abtrennt und gegebenenfalls lyophilisiert.

3. Verfahren nach Anspruch 2, dadurch gekennzeichnet, daß man das Abtrennen der Fraktion mit Molekulargewichten unter 10 000 durch Molekularfiltration durchführt.

4. Verfahren nach Anspruch 2 oder 3, dadurch gekennzeichnet, daß man als mit Wasser mischbares organisches Lösungsmittel Aceton verwendet.

5. Verfahren nach Anspruch 2 bis 4, dadurch gekennzeichnet, daß man das Abtrennen der in den flüssigen Suspensionen enthaltenen Feststoffe beziehungsweise unlöslichen Teile durch Zentrifugieren durchführt.

6. Verfahren nach Anspruch 2 bis 5, dadurch gekennzeichnet, daß man als fettlösende organische Lösungsmittel Chlorkohlenwasserstoffe verwendet.

7. Arzneimittel, gekennzeichnet durch einen Gehalt am Oligopeptid nach Anspruch 1 als Wirkstoff, zweckmäßigerweise zusammen mit üblichen pharmazeutischen Konfektionierungsmitteln.

**Patentansprüche für den Vertragsstaat: AT**

1. Verfahren zur Herstellung eines aus gesunden weißen Blutkörperchen beziehungsweise Granulozyten isolierbaren, das Wachstum beziehungsweise die Vermehrung von normalen und leukämischen myeloiden Zellen selektiv hemmenden Oligopeptides der summenmäßigen Aminosäurezusammensetzung

Tau$_1$, Asx$_1$, Ser$_2$, Thr$_1$, Glx$_3$, Gly$_2$, Ala$_1$, (PO$_4$)$_1$,

worin

Tau für einen Rest von Taurin steht,
Asx einen Rest von L-Asparagin und/oder L-Asparaginsäure bedeutet,
Ser einen Rest von L-Serin darstellt,
Thr für einen Rest von L-Threonin steht,
Glx einen Rest von L-Glutamin und/oder L-Glutaminsäure bedeutet,
Gly einen Rest von Glykokoll darstellt und
Ala für einen Rest von L-Alanin steht,

welches bei einem pH-Wert von 6,5 eine negative Ladung und eine auf Asparaginsäure bezogene relative elektrophoretische Beweglichkeit von −0,55 bis −0,65 beziehungsweise bei einem pH-Wert von 1,9 keine Ladung und eine auf ε-(Dinitrophenyl)-lysin bezogene relative Beweglichkeit von etwa 0,26 zeigt und auf Chlortolidin positiv reagiert, dadurch gekennzeichnet, daß man

a) aus tierischem oder menschlichem Blut, insbesondere Pferdeblut, gewonnene gesunde weiße Blutkörperchen in einer Pufferlösung mit einem pH-Wert von 7 bis 8 homogenisiert, das flüssige Homogenisat zentrifugiert, aus der überstehenden Flüssigkeit die gelösten Bestandteile mit Molekulargewichten unter 10 000 abtrennt, diese Fraktion einer weiteren Fraktionierung, zweckmäßig durch Gelchromatographie, unterwirft und die sich bei den Volumwerten $\frac{V_e}{V_o}$ von 1,15 bis 1,45 abscheidende Fraktion abtrennt und gegebenenfalls lyophilisiert oder

b) Granulozyten enthaltende tierische Organe, insbesondere Kalbsmilz, in einem mit Wasser mischbaren organischen Lösungsmittel homogenisiert, aus der erhaltenen flüssigen Suspension die Feststoffe abtrennt und mit einem fettlösenden organischen Lösungsmittel extrahiert, die so erhaltenen Feststoffe mit Wasser extrahiert, aus der erhaltenen flüssigen Suspension den unlöslichen Teil abtrennt und aus der flüssigen Phase die gelösten Bestandteile mit Molekulargewichten unter 10 000 abtrennt, diese Fraktion einer weiteren Fraktionierung, zweckmäßig durch Gelchromatographie, unterwirft und die sich bei den Volumwerten $\frac{V_e}{V_o}$ von 1,3 bis 2,5 abscheidende Fraktion abtrennt und gegebenenfalls lyophilisiert

und das nach einer der obigen Varianten a) und b) erhaltene Wirkstoffkonzentrat durch ein auf Grund von Ionenladung und Teilchengröße fraktionierendes Chromatograpieren und/oder durch Papier-Elektrophorese reinigt und das bei einem pH-Wert von 6,5 eine negative Ladung und eine auf Asparaginsäure bezogene relative Beweglichkeit von − 0,55 bis − 0,65 beziehungsweise bei einem pH-Wert von 1,9 keine Ladung und eine auf ε-(Dinitrophenyl)-lysin bezogene relative Beweglichkeit von etwa 0,26 zeigende und auf Chlortolidin positiv reagierende Oligopeptid abtrennt und gegebenenfalls lyophilisiert.

2. Verfahren nach Anspruch 1, dadurch gekennzeichnet, daß man das Abtrennen der Fraktion mit Molekulargewichten unter 10 000 durch Molekularfiltration durchführt.

3. Verfahren nach Anspruch 1 oder 2, dadurch gekennzeichnet, daß man als mit Wasser mischbares organisches Lösungsmittel Aceton verwendet.

4. Verfahren nach Anspruch 1 bis 3, dadurch gekennzeichnet, daß man das Abtrennen der in den flüssigen Suspensionen enthaltenen Feststoffe beziehungsweise unlöslichen Teile durch Zentrifugieren durchführt.

5. Verfahren nach Anspruch 1 bis 4, dadurch gekennzeichnet, daß man als fettlösende organische Lösungsmittel Chlorkohlenwasserstoffe verwendet.

**Claims for the Contracting states: BE, CH, DE, FR, GB, IT, LI, NL, SE**

1. Oligopeptide isolable from sound erythrocytes or granulocytes, respectively, selectively inhibiting the growth or proliferation, respectively, of normal and leukemic myeloid cells, characterized in that it has the gross amino acid composition

$Tau_1$, $Asx_1$, $Ser_2$, $Thr_1$, $Glx_3$, $Gly_2$, $Als_1$, $(PO_4)_1$

in which

Tau represents a radical of taurine,
Asx means a radical of L-asparagin and/or L-asparaginic acid,
Ser represents a radical of L-serine,
Thr means a radical of L-threonine,
Glx represents a radical of L-glutamine and/or L-glutamic acid,
Gly means a radical of glycine and
Ala represents a radical of L-alanine

and exhibits at a pH-value of 6.5 a negative charge and a relative electrophoretic mobility of from − 0.55 to − 0.65 referred to asparaginic acid or at a pH-value of 1.9 no charge and a relative mobility of about 0.26 referred to ε-(dinitrophenyl)-lysine and has a positive reaction to chlorotolidine.

2. A process for preparing the oligopeptide according to claim 1, characterized in that one

a) homogenises sound erythrocytes gained from animal or human blood, particularly horse blood, in a buffer solution having a pH-value of from 7 to 8, one centrifuges the liquid homogenisate, one separates from the supernatant liquid the dissolved components having molecular weights below 10 000, one subjects this fraction to a further fractionation, suitably by gel chromatography, and separates the fraction precipitating at volume values $\frac{V_e}{V_o}$ of from 1.15 to 1,45 and optionally lyophilises it or

b) homogenises animal organs containing granulocytes, particularly calf spleen, in an organic solvent miscible with water, one separates from the obtained liquid suspension the solids and extracts them with an organic solvent dissolving fats, one extracts the thus obtained solids with water, one separates from the liquid suspension obtained the insoluble part and one separates from the liquid phase the dissolved components having molecular weights below 10 000, one subjects this fraction to a further fractionation, suitably by gel chromatography, and one separates the fraction precipitating at volume values $\frac{V_e}{V_o}$ of from 1.3 to 2.5 and optionally lyophilises it

and one purifies the concentrate of active principle obtained according to one of the above variants a) and b) by chromatographing fractionating on account of the ionic charge and particle size and/or by paper-electrophoresis and one separates the oligopeptide having at a pH-value of 6.5 a negative charge and a relative mobility of from −0.55 to −0.65 referred to asparaginic acid or exhibiting at a pH-value of 1.9 no charge and a relative mobility of about 0.26 referred to ε-(dinitrophenyl)-lysine, respectively and positively reacting to chlorotolidine and optionally lyophilises it.

3. A process according to claim 2, characterized in that one carries out the separation of the fraction having molecular weights below 10 000 by molecular filtration.

4. A process according to claim 2 or 3, characterized in that one uses as an organic solvent miscible with water acetone.

5. A process according to claims 2 to 4, characterized in that one carries out the separation of the solids contained in the liquid suspensions or of the insoluble parts, respectively, by centrifuging.

6. A process according to claims 2 to 5, characterized in that one uses as organic solvents dissolving fats chlorinated hydrocarbons.

7. Medicaments, characterized by a contents of an oligopeptide according to claim 1 as an active principle, suitably together with usual pharmaceutical confectioning agents.

**Claims for the Contracting state: AT**

1. A process for preparing an oligopeptide isolable from sound erythrocytes or granulocytes, respectively, selectively inhibiting the growth or proliferation, respectively, of normal and leukemic myeloid cells having the gross amino acid composition

$Tau_1, Asx_1, Ser_2, Thr_1, Glx_3, Gly_2, Ala_1, (PO_4)_1$

in which

Tau represents a radical of taurine,
Asx means a radical of L-asparagin and/or L-asparaginic acid,
Ser represents a radical of L-serine,
Thr means a radical of L-threonine,
Glx represents a radical of L-glutamine and/or L-glutamic acid,
Gly means a radical of glycine and
Ala represents a radical of L-alanine

and exhibiting at a pH-value of 6.5 a negative charge and a relative electrophoretic mobility of from −0.55 to −0.65 referred to asparaginic acid or at a pH-value of 1.9 no charge and a relative mobility of about 0.26 referred to ε-(dinitrophenyl)-lysine and having a positive reaction to chlorotolidine, characterized in that one

a) homogenises sound erythrocytes gained from animal or human blood, particularly horse blood, in a buffer solution having a pH-value of from 7 to 8, one centrifuges the liquid homogenisate, one separates from the supernantant liquid the dissolved components having molecular weights below 10 000, one subjects this fraction to a further fractionation, suitably by gel chromatography, and separates the fraction precipitating at volume values $\frac{V_e}{V_o}$ of from 1.15 to 1.45 and optionally lyophilises it or

b) homogenises animal organs containing granulocytes, particularly calf spleen, in an organic solvent miscible with water, one separates from the obtained liquid suspension the solids and extracts them with an organic solvent dissolving fats, one extracts the thus obtained solids with water, one separates from the liquid suspension obtained the insoluble part and one separates from the liquid phase the dissolved components having molecular weights below 10 000, one subjects this fraction to a further fractionation, suitably by gel chromatography, and one separates the fraction precipitating at volume values $\frac{V_e}{V_o}$ of from 1.3 to 2.5 and optionally lyophilises it

and one purifies the concentrate of active principle obtained according to one of the above variants a) and b) by chromatographing fractionating on account of the ionic charge and particle size and/or by paper-electrophoresis and one separates the oligopeptide having at a pH-value of 6.5 a negative charge and a relative mobility of from −0.55 to −0.65 referred to asparaginic acid or exhibiting at a pH-value of 1.9 no charge and a relative mobility of about 0.26 referred to ε-(dinitrophenyl)-lysine, respectively and positively reacting to chlorotolidine and optionally lyophilises it.

2. A process according to claim 1, characterized in that one carries out the separation of the fraction having molecular weights below 10 000 by molecular filtration.

3. A process according to claim 1 or 2, characterized in that one uses as an organic solvent miscible with water acetone.

4. A process according to claims 1 to 3, characterized in that one carries out the separation of the solids contained in the liquid suspensions or of the insoluble parts, respectively, by centrifuging.

5. A process according to claims 1 to 4, characterized in that one uses as organic solvents dissolving fats chlorinated hydrocarbons.


**Revendications pour les Etats contractants: BE, CH, DE, FR, GB, IT, LI, NL, SE**

1. Oligopeptide isolable à partir de globules blancs sains ou de granulocytes, inhibant sélectivement la croissance ou la multiplication, respectivement, de cellules myéloïdes normales et leucémiques et qui est caractérisé par le fait qu'il présente la composition globale en aminoacides:

$$Tau_1, Asx_1, Ser_2, Thr_1, Glx_3, Gly_2, Ala_1, (PO_4)_1,$$

dans laquelle

Tau représente un radical taurine,
Asx représente un radical L-asparagine et/ou acide L-aspartique,
Ser représente un radical L-sérine,
Thr représente un radical L-thréonine,
Glx représente un radical L-glutamine et/ou acide L-glutamique,
Gly représente un radical glycocolle et
Ala représente un radical L-alanine,

et qu'il présente, à un pH de 6,5, une charge négative et une mobilité électrophorétique relative, rapportée à l'acide aspartique, de −0,55 à −0,65 et ne présente à un pH de 1,9 aucune charge et présente une mobilité relative, rapportée à l'ε-(dinitrophényl)-lysine, d'environ 0,26 et réagit positivement sur la chlorotolidine.

2. Procédé de préparation de l'oligopeptide selon la revendication 1, caractérisé en ce que:

a) l'homogénéise des globules blancs sains tirés de sang animal ou humain, en particulier de sang de cheval, dans une solution tampon ayant un pH de 7 à 8, on centrifuge le produit d'homogénéisation liquide, on sépare du liqueur surnageant, les constituants dissous ayant des poids moléculaires inférieurs à 10 000, on soumet cette fraction à un nouveau fractionnement, avantageusement par chromatographie sur gel et on isole la fraction qui se sépare aux valeurs de

volume $\frac{V_e}{V_o}$ de 1,15 à 1,45 et éventuellement on la lyophilise, ou,

b) l'on homogénéise des organes animaux contenant des granulocytes, en particulier de la rate de veau, dans un solvant organique miscible à l'eau, on sépare les solides de la suspension liquide obtenue et on les extrait par un solvant organique dissolvant les graisses, on extrait par l'eau les solides ainsi obtenus, on sépare de la suspension liquide obtenue la partie insoluble et on sépare de la phase liquide les constituants dissous ayant des poids moléculaires inférieurs à 10 000, on soumet cette fraction à un nouveau fractionnement, avantageusement par chromatographie sur gel et on isole la fraction qui se sépare aux valeurs de volume $\frac{V_e}{V_o}$ de 1,3 à 2,5 et éventuellement on la lyophilise,

et l'on purifie le concentré de substance active, obtenu selon l'une des variantes a) et b) ci-dessus, par une chromatographie assurant le fractionnement sur la base de la charge ionique et de la grosseur de particules et/ou par électrophorèse sur papier et on sépare l'oligopeptide qui présente à un pH de 6,5, une charge négative et une mobilité relative, rapportée à l'acide aspartique, de −0,55 à −0,65 ou qui, à un pH de 1,9 ne présente aucune charge et présente une mobilité relative, rapportée à l'ε-(dinitrophényl)-lysine, d'environ 0,26 et réagit positivement sur la chlorotolidine, et on le lyophilise éventuellement.

3. Procédé selon la revendication 2, caractérisé en ce que l'on effectue la séparation de la fraction ayant des poids moléculaires inférieurs à 10 000 par filtration moléculaire.

4. Procédé selon la revendication 2 ou 3, caractérisé en ce qu'on utilise l'acétone comme solvant organique miscible à l'eau.

5. Procédé selon l'une quelconque des revendications 2 à 4, caractérisé en ce que l'on réalise la séparation des solides ou des fractions insolubles contenus dans les suspensions liquides, par centrifugation.

6. Procédé selon l'une quelconque des revendications 2 à 5, caractérisé en ce qu'on utilise un hydrocarbure chloré comme solvant organique dissolvant les graisses.

7. Médicament caractérisé en ce qu'il contient l'oligopeptide selon la revendication 1, comme substance active, avantageusement associé à des agents de conditionnement pharmaceutiques usuels.

## Revendications pour l'Etat contractant: AT

1. Procédé de préparation d'une oligopeptide isolable à partir de globules blancs sains ou de granulocytes, inhibant sélectivement la croissance ou la multiplication, respectivement, de cellules myéloïdes normales et leucémiques et présentant la composition globale en aminoacides:

$$Tau_1, Asx_1, Ser_2, Thr_1, Glx_3, Gly_2, Ala_1, (PO_4)_1,$$

dans laquelle

Tau représente un radical taurine,
Asx représente un radical L-asparagine et/ou acide L-aspartique,
Ser représente un radical L-sérine,
Thr représente un radical L-thréonine,
Glx représente un radical L-glutamine et/ou acide L-glutamique,
Gly représente un radical glycocolle et
Ala représente un radical L-alanine,

qui présente, à un pH de 6,5, une charge négative et une mobilité électrophorétique relative, rapportée à l'acide aspartique, de −0,55 à −0,65 et ne présente à un pH de 1,9 aucune charge et présente une mobilité relative, rapportée à l'ε-(dinitrophényl)-lysine, d'environ 0,26 et réagit positivement sur la chlorotolidine, caractérisé en ce que:

a) l'homogénéise des globules blancs sains tirés de sang animal ou humain, en particulier de sang de cheval, dans une solution tampon ayant un pH de 7 à 8, on centrifuge le produit d'homogénéisation liquide, on sépare du liqueur surnageant, les constituants dissous ayant des poids moléculaires inférieurs à 10 000, on soumet cette fraction à un nouveau fractionnement, avantageusement par chromatographie sur gel et on isole la fraction qui se sépare aux valeurs de

volume $\frac{V_e}{V_o}$ de 1,15 à 1,45, et éventuellement on la lyophilise, ou,

b) l'on homogénéise des organes animaux contenant des granulocytes, en particulier de la rate de veau, dans un solvant organique miscible à l'eau, on sépare les solides de la suspension liquide obtenue et on les extrait par un solvant organique dissolvant les graisses, on extrait par l'eau les solides ainsi obtenus, on sépare de la suspension liquide obtenue la partie insoluble et on sépare de la phase liquide les constituants dissous ayant des poids moléculaires inférieurs à 10 000, on soumet cette fraction à un nouveau fractionnement, avantageusement par chromatographie sur gel et on isole la fraction qui se sépare aux valeurs de volume $\frac{V_e}{V_o}$ de 1,3 à 2,5 et éventuellement on la lyophilise,

et l'on purifie le concentré de substance active, obtenu selon l'une des variantes a) et b) ci-dessus, par une chromatographie assurant le fractionnement sur la base de la charge ionique et de la grosseur de particules et/ou par électrophorèse sur papier et on sépare l'oligopeptide qui présente à un pH de 6,5, une charge négative et une mobilité relative, rapportée à l'acide aspartique, de −0,55 à −0,65 ou qui, à un pH de 1,9 ne présente aucune charge et présente une mobilité relative, rapportée à l'ε-(dinitrophényl)-lysine, d'environ 0,26 et réagit positivement sur la chlorotolidine, et on le lyophilise éventuellement.

2. Procédé selon la revendication 1, caractérisé en ce que l'on effectue la séparation de la fraction ayant des poids moléculaires inférieurs à 10 000 par filtration moléculaire.

3. Procédé selon la revendication 1 ou 2, caractérisé en ce qu'on utilise l'acétone comme solvant organique miscible à l'eau.

4. Procédé selon l'une quelconque des revendications 1 à 3, caractérisé en ce que l'on réalise la séparation des solides ou des fractions insolubles contenus dans les suspensions liquides, par centrifugation.

5. Procédé selon l'une quelconque des revendications 1 à 4, caractérisé en ce qu'on utilise un hydrocarbure chloré comme solvant organique dissolvant les graisses.